# EUROPEAN PATENT APPLICATION

(11) **EP 1 505 055 A1**
(43) Date of publication of application: **09.02.2005**
(21) Application number: 04254649.9
(22) Date of filing: 02.08.2004
(51) Int. Cl.: C07C 69/88, C11B 9/00, A61K 7/46

(54) **Fragrance compositions**

(30) Priority: 06.08.2003 US 635356
(71) Applicant: INTERNATIONAL FLAVORS & FRAGRANCES INC., New York New York 10019 (US)
(72) Inventor: Narula, Anubhav P. S., Hazlet New Jersey 07730 (US)
(74) Representative: Mercer, Christopher Paul

(57) **Abstract**

The use of salicylate compounds as a fragrance chemical, suitable for use in creating fragrance in items such as perfumes, colognes and personal care products is disclosed.

## Description

### Field of the Invention

Novel salicylate compounds are disclosed as well as the use of these salicylate compounds as fragrance chemicals suitable for incorporation in fine fragrances, cosmetics, toiletries and related applications.

### Background of the Invention

There is an ongoing need in the fragrance industry to provide new chemicals to give perfumers, formulators and other persons in the perfumery field the ability to create new fragrances for perfumes, colognes and personal care products.

Salicylate materials are currently sold as fragrance chemicals and include materials such as benzyl salicylate, cis-hexenyl salicylate and cyclohexexyl salicylate. Despite the prior use of salicylate materials there is an ongoing need for the discovery of new materials that can be used as fragrance materials in functional perfumery.

### Summary of the Invention

The present invention is directed to the novel compounds having the formula set forth below: 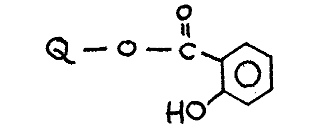 where Q is selected from R¹, R² and R³;
R¹ is understood to be 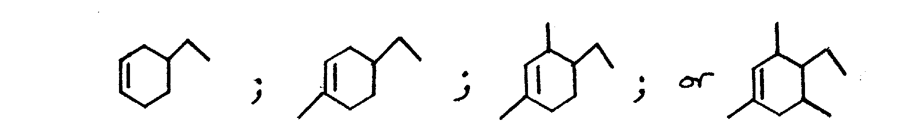 R² is understood to be 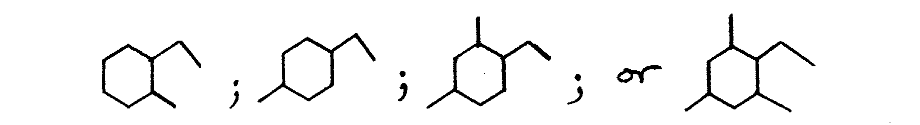 and R³ is understood to be

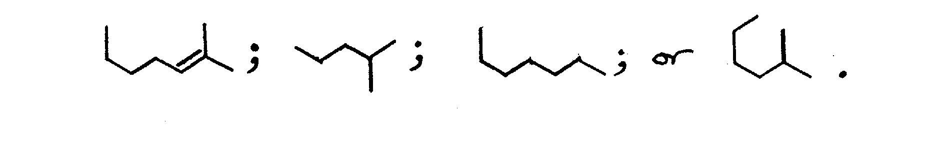

The present invention is also directed to a method of using the compounds of the formulas set forth above as well as the compound: 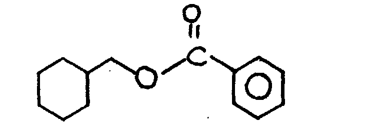 as a fragrance chemical to enhance fragrance in perfumes, toilet waters, colognes, personal products and the like.

These and other embodiments of the present invention will be apparent by reading the following specification.

### Detailed Description of the Invention

The present invention is directed to the above described novel compounds and the use of these compounds as fragrance chemicals.

We have discovered that these salicylate compounds have a green, floral odor or note, that are well suited for use as fragrance chemicals.

These compounds are prepared by the reaction of salicylic acid with the appropriate alcohol. Suitable alcohols for carrying out the esterification reactions are set forth in the Examples below. The reactions are preferably conducted in a solvent and catalyst, with toluene being the preferred solvent and preferred catalysts are para-tolune sulfonic acid (p-TSA), potassium carbonate and mono butyl tin oxide. After washing the product, usually with water, the salicylate compounds of the present invention are recovered. Further description of the reactions used to manufacture the products are set forth in the Examples below.

The use of these compounds are widely applicable in current perfumery products, including the preparation of perfumes and colognes, the perfuming of personal care products such as soaps, shower gels, and hair care products such as shampoos, rinse conditioners, coloring compositions and the like; as well as air fresheners, candles and cosmetic products. The compound can also be used to perfume candles and cleaning agents, such as, but not limited to soaps, detergents, dishwashing materials, scrubbing compositions, window cleaners, and the like.

In these preparations, the compound of the present invention can be used alone or in combination with other fragrance compositions, solvents, adjuvants and the like. Those with skill in the art will appreciate the nature and variety of the other ingredients that can be used in combination with the compound of the present invention.

Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, and carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in U.S. Patent No. 4,534,891. Another source of suitable fragrances is found in Perfumes, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

As used herein olfactory effective amount is understood to mean the amount of compound in perfume compositions the individual component will contribute to its particular olfactory characteristics, but the olfactory effect of the perfume composition will be the sum of the effects of each of the perfume or fragrance ingredients. Thus the compounds of the invention can be used to alter the aroma characteristics of the perfume composition by modifying the olfactory reaction contributed by another ingredient in the composition. The amount will vary depending on many factors including other ingredients, their relative amounts and the effect that is desired.

The level of compound of the invention employed in the perfumed article varies from about 0.005 to about 20 weight percent, preferably from about 0.1 to about 10 and most preferably from about 0.5 to about 5 weight percent. In addition to the compounds, other agents can be used in conjunction with the fragrance. Well known materials such as surfactants, emulsifiers, and polymers to encapsulate the fragrance can also be employed without departing from the scope of the present invention.

Another method of reporting the level of the compound of the invention in the perfumed composition, i.e., the compounds as a weight percentage of the materials added to impart the desired fragrance. The compounds of the invention can range widely from 0.005 to about 10 weight percent of the perfumed composition, and preferably from about 0.1 to about 5 weight percent. Those with skill in the art will be able to employ the desired level of the compound of the invention to provide the desired fragrance and intensity.

The following are provided as specific embodiments of the present invention. Other modifications of this invention will be readily apparent to those skilled in the art, without departing from the scope of this invention. As used herein all percentages are weight percent. IFF as used in the examples is understood to mean International Flavors & Fragrances Inc., New York, N.Y.; DPG is understood to mean dipropylene glycol, DEP is understood to mean diethylphthalate.

### EXAMPLE 1

### Preparation of benzoic acid, 2-hydroxy-(2,4,6-trimethyl-3-cyclohexen-1-yl)methyl ester

To a 3-liter flask fitted with Bidwell condenser, mixer and under a nitrogen blanket, 1200 grams of iscyclogeraniol, 984 grams of salicyclic acid, 400 grams toluene and 2 grams of p-TSA were charged. The contents were refluxed at a temperature of about 150°C. After being held for about 10 hours and the removal of about 100 milliliters of water, the product was recovered. The boiling point was about 128°C at 0.6 mm. The spectra of the compound is as follows 00.92-1.13(ms, 6H), 1.7(s,3H), 1.8-2.48(m,4H), 4.3(M,1H), 4.5(s, 1H), 5.2(d,1H), 6.8-7.8(4m,4H).

The product was found to have a spicy geraniol note.

### EXAMPLE 2

### Preparation of salicyclic acid (2,4-dimethyl-3-cyclohexen-1-yl) methyl ester

Using similar equipment as used in Example 1 above, 567 grams of FLORALOL [IFF], 466 grams of salicyclic acid, 200 grams of toluene and 1.0 grams of p-TSA were charged. The ingredients were heated to reflux and water was collected. The reaction was allowed to proceed for about 10 hours at 140°C and about 170 milliliters of solvent was removed. The product had a boiling point of 156°C at 2.2 mm. The spectra of the compound is 0.9-1.1 (ms,3H), 1.8-2.4(m,5H), 4.2(m,2H), 5.2(d,1H), 6.8-7.8(4m,4H).

The product was found to have a green salicylate note.

### EXAMPLE 3

### Preparation of salicyclic acid (3-cyclohexen-1-yl)methyl ester

Using the equipment set forth in Example 1, 380 grams of 3-cyclohexanetetrahydrobenzyl alcohol, 497 grams of salicyclic acid, 300 milliliters of toluene and 8 grams of mono butyl tin oxide catalyst was charged and heated to about 140°C. The contents were allowed to reflux for about 20 hours and about 120 milliliters of water were removed.

The product was found to have a note similar to benzyl salicylate.

### EXAMPLE 4

### Preparation of salicylic acid (4-methyl-3-cyclohexen-1-yl)methyl ester

Using the equipment described in Example 1, 465 grams of para- methyl -3-cyclohexanetetrahydrobenzyl alcohol, 393 grams of salicyclic acid, 3 grams of mono butyl tin oxide and 25 grams of toluene were charged and heated to reflux at about 180°C. The contents were allowed to reflux for about 6 hours and about 150 milliliters were removed. The product had a boiling point of 145°C at 0.6 mm. The spectra of the compound is as follows: 1.5-2.1(m and s, 7H), 4.2(s, 2H), 5.3(d,1H), 6.8-7.8(4m,4H).

The odor of the product was described as green and having aubepine notes.

### EXAMPLE 5

### Preparation of Salicylic acid (2,4,6-trimethylcyclohexyl)methyl ester

Using the equipment of Example 1, 508 grams of DIHYDROISOCYCLOGERANIOL [IFF], 378 grams of salicyclic acid, 200 grams of toluene and 1 grams of p-toluene sulfonic acid were charged and heated to about 140°C to reflux. The contents were allowed to reflux for about 20 hours and a total of 80 milliliters of water was removed.

The product was washed with 500 milliliters of 5% NaHCO₃ and agitated for 20 minutes and washed twice with 600 milliliters NaHCO₃ and once with 600 milliliters of saturated NaCl in water. The spectra of the product is as follows: 0.7-1.1(ms,9H), 1.2-2.2(m,8H), 4.2-4.5(m, 2H),6.8-7.8(4m,4H).

The product had a spicy, green, isocyclogeraniol note.

### EXAMPLE 6

### Preparation of Salicylic acid (2,4-dimethylcyclohexyl)methyl ester

Using the equipment of Example 1, 520 grams of DIHYDROFLOROLOL [IFF], 425 grams of salicyclic acid, 200 milliliters of toluene and 1 gram of p-TSA. The contents were heated to reflux to about 150°C and about 130 milliliters of water was removed over about 25 hours.

The resulting product was washed with 800 milliliters of 5% NaHCO₃ and two washes of 700 milliliters of 5% NaHCO₃ followed by a single wash of saturated NaCl. The spectra of the product is as follows: 0.85-1.0(ms,6H), 1.3-2.0(m,12H), 2.25-2.5(m,1H), 4.0-4.2(m,2H), 6.8-7.8(4m,4H).

The product had a green, floral salicylate note.

### EXAMPLE 7

### Preparation of Salicylic acid (4-methylcyclohexyl) methyl ester

Using the equipment described in Example 1, 465 grams of para-methylhexhydrobenzyl alcohol, 393 grams of salicyclic acid, 3 grams of p-TSA and 25 grams of toluene were charged and heated to reflux at about 180 °C. The contents were allowed to reflux for about 6 hours and about 150 milliliters were removed. The product had a boiling point of 145°C at 0.6 mm.

The odor of the product was described as green and having aubepine notes.

### EXAMPLE 8

### Preparation of Salicylic acid 1,2-dimethyl butyl ester

Methyl salicyclic [487 grams], 464 grams of 3-methyl-2-pentyl alcohol, and 12.4 grams of mono butyl tin oxide were charged to equipment as described in Example 1. The contents were brought to reflux. As distillation was initiated, 0.8 milliters per minute were removed. The contents were allowed to reflux over night.

The contents were cooled and the catalyst was filtered and rinsed with water. The product had a fragrance that had a sassafras note. The spectra of the product was as follows: 0.9 -1.05(d, 6H), 1.3(d,3H), 1.6(s,1H), 5.1(m, 1H), 6.9-7.85(4m,4H).

### EXAMPLE 9

### Fragrance Formulation

The following fragrance formulation was prepared:

| | |
|---|---|
| C-11 Aldehyde Ulenic [Firmenich] | 0.60 grams |
| C-12 Aldehyde Lauric | 0.50 |
| C-9 Aldehyde | 1.30 |
| Benzyl Acetate | 0.10 |
| Benzyl Isovalerate | 3.00 |
| Benzyl Salicylate | 55.00 |
| Dihydolinalool | 0.60 |
| Delta dodecalactone | 14.00 |
| Ethyl benzoate | 7.00 |
| Eugenol trubeck | 4.50 |
| Geranyl acetone | 18.00 |
| Indol in 10% DPG | 60.00 |
| Isobutyl benzoate | 5.00 |
| Jasmolactone [Firmenich] | 42.00 |
| Kharismal | 230.00 |
| Linalool | 0.90 |
| Methyl anisate | 110.00 |
| Methyl benzoate | 21.50 |
| Methyl cinnamate | 24.50 |
| Methyl para cresol | 2.60 |
| Nerolidol | 397.60 |
| Total | 998.70 |

1.3 grams of salicylic acid, 1,2-dimethylbutyl ester was added to the above formulation. The addition of the salicylic acid, 1,2-dimethylbutyl ester was found to add character and purity to the creation of this floral scent.

### EXAMPLE 10

### Fragrance Formulation

| | |
|---|---|
| Iso cylco geraniol | 665.00 grams |
| Cinnaminic alcohol | 40.00 |
| Citronella | 25.00 |
| Geraniol Coeur | 35.00 |
| Piperonal | 25.00 |
| Phenyl Ethyl Alcohol | 65.00 |
| Phenyl Propyl Alcohol | 5.00 |
| Ylang [Extra Pure] | 8.0 |
| Total | 868.00 |

132 grams of salicylic acid 1,2-methyl butyl ester was added to the above fragrance formulation to provide a spicy note in the fragrance.

### EXAMPLE 11

### Preparation of Salicyclic Acid: 1,5-dimethyl-4-hexenyl ester

Methyl salicylate [796 grams], 6-methyl-5-hepten-2-ol [505 grams], 14 grams of potassium carbonate and 1580 grams of toluene were charged under nitrogen to equipment as described in Example 1 above. The contents were heated to reflux and were maintained at a temperature of about 120 - 130 °C. Distillate was periodically removed while the contents were being refluxed. A total of about 1000 milliliters of distillate was removed.

After the allowing the contents to reflux for about 24 hours, about 700 mls of 5% solution of AcOH was added dropwise to the vessel. The contents were agitated for 20 minutes and then allowed to separate. The contents were washed with 2x 700 milliters of 5%AcOH and 2x 800 milliliters of saturated NaCl in water.

The product was reported to have a green, salicylate odor, with floral notes.

### EXAMPLE 12

### Preparation of Salicyclic acid:1,4-dimethylpentyl ester

5-methyl-2 hexanol [515 grams], methyl salicylate [875 grams], potassium carbonate [15 grams] and 400 milliliters of toluene were charged to equipment described in Example 1. The contents were held at reflux, 120-130 °C. The contents were allowed to reflux for about 24 hours over 2 days, before cooling.

The resulting product had a green salicylate fragrance, with floral fruity notes.

### EXAMPLE 13

### Preparation of Salicyclic acid:1-methylhexyl ester

2-heptanol [530 grams], methyl salicylate [562 grams], potassium carbonate [16 grams] and toluene [270 milliliters] were charged to equipment described in Example 1. The contents were heated to about 140 °C and allowed to reflux for about 20 hours while occasionally removing some solvent.

The product was cleaned with water washes and recovered. The product had a low key salicylate fragrance with smokey notes.

### EXAMPLE 14

### Preparation of Salicylic acid: 1-ethyl-3-methylpentylester

5-methyl-2-heptanol [780 grams], methyl salicylate [514 grams], potassium carbonate [15 grams] and mesitylene [420 milliliters] were charged to equipment described in Example 1. The contents were heated to about 155 °C and allowed to reflux for about 20 hours while occasionally removing some solvent.

The product was cleaned with water washes and recovered. The product had a salicylate and green, floral fragrance with chocolate and fruity notes.

## Claims

1. The compound of the formula 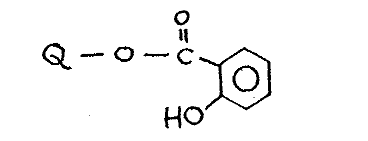 wherein
Q is selected from R¹ , R² and R³ and
R¹ is understood to be 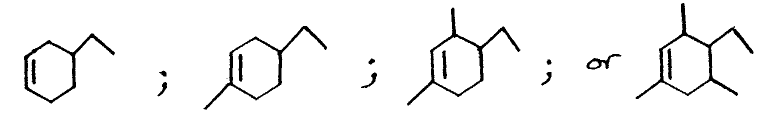 R² is understrood to be 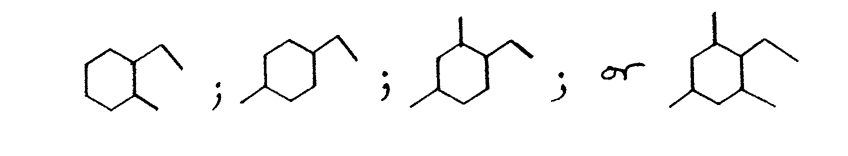 and R³ is understood to be

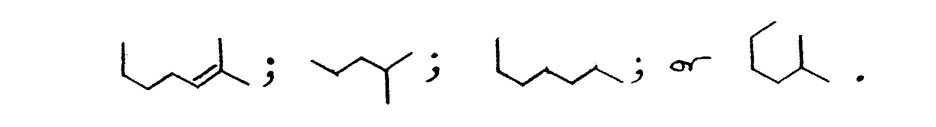

2. The compound claim 1 wherein R¹ is:

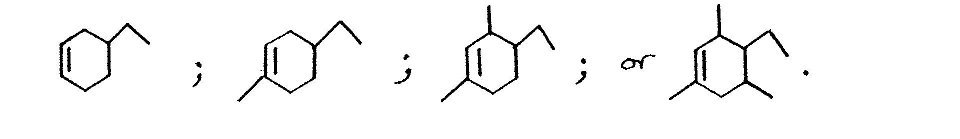

3. The compound of claim 1 or claim 2 wherein R² is:

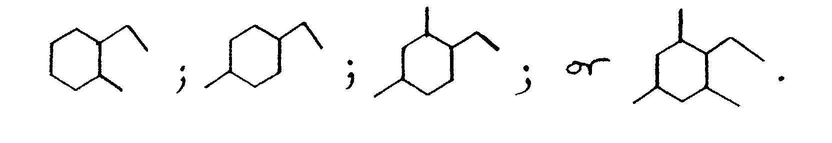

4. The compound of any one of claims 1 to 3 wherein R³ is:

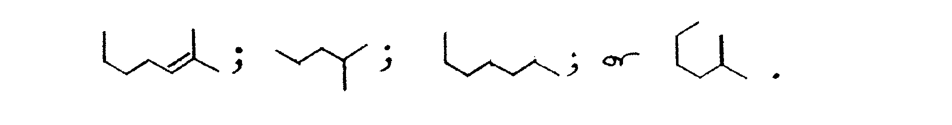

5. A fragrance composition containing an olfactory acceptable amount of a compound of any one of claims 1 to 4 and

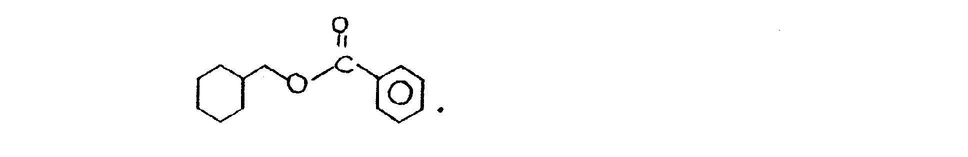

6. The fragrance composition of claim 5 which is incorporated into a perfume, cologne, candle, toilet water, cosmetic product, personal care product, fabric care product, cleaning product or air freshener.

7. A method for improving, enhancing or modifying a fragrance through the addition of an olfactory acceptable amount of a compound of any one of claims 1 to 4 and

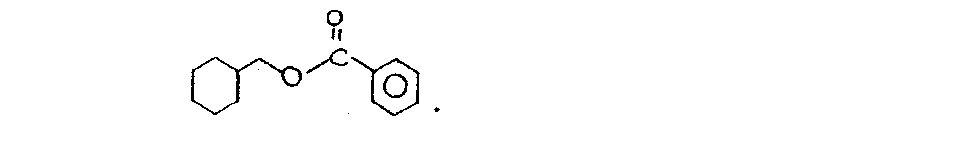

8. The method of claim 7 wherein the fragrance is incorporated into a perfume, cologne, candle, toilet water, cosmetic product, personal care product, fabric care product, cleaning product or air fresheners.

9. The method of claim 8 wherein the cleaning product is a soap, detergent, dishwashing composition, scrubbing compound or window cleaner.

10. The method of claim 8 wherein the product is a personal care product.

11. The method of claim 7 wherein the olfactory acceptable level is from 0.005 to 20 weight percent, preferably 0.1 to 10 weight percent, more preferably 0.5 to 5 weight percent.
